(19) Europäisches Patentamt
European Patent Office
Office européen des brevets

(11) **EP 2 289 945 B1**

(12) **FASCICULE DE BREVET EUROPEEN**

(45) Date de publication et mention de la délivrance du brevet:
**04.03.2015 Bulletin 2015/10**

(51) Int Cl.:
*A61L 27/50* *(2006.01)*  *A61L 27/20* *(2006.01)*
*C08B 37/08* *(2006.01)*  *C08J 3/24* *(2006.01)*
*A61L 27/52* *(2006.01)*

(21) Numéro de dépôt: **10181594.2**

(22) Date de dépôt: **08.04.2004**

(54) **Réticulation de hyaluronates de sodium de faible et forte masse moléculaire; préparation d'hydrogels monophasiques injectables; hyaluronate de sodium rétioculé et hydrogel ainsi obtenus**

Vernetzung von Natriumhyaluronaten mit niedrigem und hohem Molekulargewicht, Herstellung von einspritzbaren einzelphasigen Hydrogelen, sowie damit hergestellte vernetztes Natriumhyaluronat und Hydrogel

Crosslinking of sodium hyaluronates with low and high molecular weight, manufacture of injectable single-phase hydrogels and crosslinked sodium hyaluronate and hydrogel thus obtained

(84) Etats contractants désignés:
**DE ES FR GB IT**

(30) Priorité: **10.04.2003  FR 0304444**

(43) Date de publication de la demande:
**02.03.2011  Bulletin 2011/09**

(60) Demande divisionnaire:
**14200048.8**

(62) Numéro(s) de document de la (des) demande(s) initiale(s) en application de l'article 76 CBE:
**04742458.5 / 1 611 160**

(73) Titulaire: **ALLERGAN INDUSTRIE**
**74370 Pringy (FR)**

(72) Inventeur: **Lebreton, Pierre**
**74000, Annecy (FR)**

(74) Mandataire: **Hoffmann Eitle**
**Patent- und Rechtsanwälte PartmbB**
**Arabellastraße 30**
**81925 München (DE)**

(56) Documents cités:
CA-A- 949 965    US-A- 4 582 865
US-A- 4 886 787    US-A- 5 531 716

• **DATABASE BIOSIS BIOSCIENCES INFORMATION SERVICE, PHILADELPHIA, PA, US; Accession number PREV200300142886 LAMAR, P. D. ET AL.: "Antifibrosis Effect of Novel Gels in Anterior Ciliary Slerotomy (ACS)", XP002265551, & LAMAR, P. D. ET AL.: "Antifibrosis Effect of Novel Gels in Anterior Ciliary Slerotomy (ACS)", ARVO ANNUAL MEETING ABSTRACT AND SEARCH PLANNER, ABSTRACT NO. 1115, vol. 2002, 2002,**

EP 2 289 945 B1

## Description

La présente invention a pour objet :

un procédé de réticulation de hyaluronate de sodium (NaHA),
- un procédé de préparation d'un hydrogel monophasique injectable d' un tel polymère,
- les polymères réticulés et hydrogels monophasiques injectables, susceptibles d'être respectivement obtenus par chacun desdits procédés.

Les hydrogels en cause, à base desdits polymères de NaHA réticulés, ont de nombreux débouchés, notamment comme matériau de comblement en chirurgies réparatrice, esthétique, dentaire, en ophtalmologie et en orthopédie comme produit empêchant les adhésions de tissus, en chirurgie générale, en urologie. Lesdits hydrogels conviennent notamment pour la réparation des cordes vocales. Les débouchés indiqués ci-dessus, de façon nullement limitative, pour ce type de produits, sont familiers à l'homme du métier.

L'invention résulte d'un réel effort d'optimisation de la mise en oeuvre de la réticulation des polymères en cause, en vue d'obtenir des hydrogels monophasiques injectables, particulièrement intéressants en référence au compromis ci-après : propriétés mécaniques et rémanence, d'une part, injectabilité (avec des forces d'injection et des diamètres d'aiguilles d'injection, acceptables), d'autre part.

On précise ici que le qualificatif "injectable", employé dans le présent texte, tant en référence aux hydrogels de l'art antérieur qu'aux hydrogels de l'invention, signifie injectable manuellement au moyen de seringues munies d'aiguilles classiques (d'un diamètre compris entre 0,1 et 0,5 mm). Dans le cadre de la présente invention, il est notamment possible de formuler des hydrogels injectables au travers d'aiguilles hypodermiques de 30 G½, 27 G½, 26 G½, 25 G.

Selon l'art antérieur, on a déjà préparé, à partir de polysaccharides et de leurs dérivés - notamment à partir de sels d'acide hyaluronique - pas, peu ou fortement réticulés, des hydrogels, notamment des hydrogels injectables.

En référence au problème spécifique de l'injectabilité, des compositions biphasiques ont été proposées, dont notamment la phase continue est à base de tels hydrogels. Ladite phase continue sert de plastifiant, de véhicule d'injection à une phase dispersée. Cette phase dispersée est plus ou moins solide, plus ou moins différenciée de la phase continue. Ainsi :

- dans la demande EP-A-0 466 300, les compositions biphasiques décrites sont constituées de deux phases - continue, dispersée - biorésorbables et se présentent sous la forme de boues. Lesdites deux phases sont avantageusement préparées à partir de fibres de Hylan (acide hyaluronique naturel modifié chimiquement in situ dans le but de faciliter son extraction des tissus),
- dans la demande WO-A-96 337 51, les compositions biphasiques décrites présentent également deux phases biorésorbables, mieux séparées, la phase dispersée étant constituée de fragments insolubles d'un hydrogel de polymère (choisi parmi l'acide hyaluronique et ses sels), fortement réticulé ;
- dans la demande WO-A-00 014 28, les compositions biphasiques décrites renferment une phase dispersée, non biorésorbable (particules d'au moins un hydrogel d'un (co)polymère obtenu par polymérisation et réticulation de l'acide acrylique et/ou de l'acide méthacrylique et/ou d'au moins un dérivé desdits acides), en suspension dans une solution aqueuse d'un polymère choisi parmi les protéines, les polysaccharides et leurs dérivés, réticulé ou non.

Ces systèmes biphasiques ne sont pas pleinement satisfaisants dans la mesure où l'on peut justement craindre des à-coups à l'injection et surtout après injection, une disparition plus rapide de la phase continue (pas ou faiblement réticulée) et donc une perte au moins partielle de l'effet, notamment de comblement, recherché.

On a donc également proposé, en parallèle, des hydrogels monophasiques, élaborés à partir des mêmes types de polymères.

Dans les demandes WO-A-98 356 39 et WO-A-98 356 40, le produit en cause n'est pas un hydrogel injectable mais un produit de consistance solide. Dans lesdites demandes, on décrit en fait des implants oculaires, utilisés pour combler temporairement un vide créé chirurgicalement. Dans le brevet US-A-4 716 154, l'hydrogel élaboré est proposé comme substitut au corps vitré. Le polymère en cause (hyaluronate de sodium) est très peu réticulé, pour l'obtention d'un hydrogel injectable. Dans la demande WO-A-02 057 53, l'hydrogel monophasique décrit est chargé d'un antiseptique, efficace pour le protéger, après implantation, vis-à-vis des radicaux libres. Dans la demande WO-A-02 063 50, un procédé est décrit, apte à générer ce type d'hydrogel, très homogène dans sa masse.

Tous ces hydrogels monophasiques ont été obtenus à partir de polymères de forte masse moléculaire, réticulés avec intervention d'une quantité efficace et non excessive d'au moins un agent de réticulation, en solvant aqueux.

Au vu de cet art antérieur, les inventeurs ont souhaité améliorer l'efficacité de la réticulation du polymère en cause de façon à notamment améliorer la résistance à la dégradation (la rémanence) de l'hydrogel implanté, tout en conservant la possibilité d'injecter ledit hydrogel dans des conditions acceptables.

Pour améliorer l'efficacité de la réticulation, les inventeurs ont songé, dans un premier temps, à faire intervenir plus d'agent de réticulation. Cette approche a rapidement été écartée dans la mesure où elle entraîne inéluctablement une dénaturation du polymère en cause et une pollution chimique du produit réticulé obtenu.

Lesdits inventeurs ont alors songé à augmenter la concentration de polymère dans le mélange réactionnel. Cette seconde approche a, de la même façon dû, a priori, être écartée à la considération des polymères utilisés classiquement à ce jour : polymères de forte masse moléculaire. Ainsi, pour le hyaluronate de sodium, il est toujours utilisé à de fortes masses (Mw $\geq 10^6$ Da, $\approx 2.10^6$ Da, $3.10^6$ Da), à des concentrations voisines de la concentration maximale qui est d'environ 105-110 mg/g. L'utiliser à plus forte concentration est difficile (la viscosité du mélange réactionnel devient trop élevée) et entraîne inéluctablement des problèmes de solubilité, de mauvaise homogénéité.

Concentrer le milieu réactionnel s'avère en revanche possible avec des polymères de faible masse moléculaire (du hyaluronate de sodium de masse moléculaire 300 000 Da, présentant une viscosité intrinsèque de 600 ml/g (l'homme du métier connaît parfaitement la relation entre ces deux paramètres : masse moléculaire (M) et viscosité intrinsèque ($\eta$), relation donnée par la formule de Mark-Houwink : $M = k \eta^{\alpha}$, k et a présentant des valeurs qui dépendent de la nature du polymère en cause) peut être concentré de 110 à 200 mg/g). Malheureusement, le polymère réticulé obtenu génère dans ces conditions un hydrogel injectable non homogène, biphasique.

Dans un tel contexte, les inventeurs ont, de manière surprenante, établi qu'en associant un polymère de NaHA de faible masse moléculaire et un polymère de NaHA de forte masse moléculaire, on obtient un excellent compromis, à savoir la possibilité de générer, pour un taux de réticulation non excessif (équivalent à celui de l'art antérieur) un hydrogel monophasique, injectable avec des propriétés mécaniques et de rémanence améliorées. Cette association faible masse moléculaire /forte masse moléculaire permet d'obtenir un hydrogel répondant de manière plus que satisfaisante au cahier des charges ci-après :

- monophasique,
- propriétés mécaniques et rémanence meilleures que celles des produits équivalents de l'art antérieur,
- injectabilité non affectée, voire améliorée, restant possible avec des forces d'injection classiques au moyen de dispositifs d'injection classiques.

Le facteur clé du procédé de réticulation de l'invention réside donc dans la concentration des réactifs (qui est augmentée par rapport à celle des mélanges réactionnels de l'art antérieur, du fait de l'intervention de polymère(s) de faible masse moléculaire), la réticulation desdits réactifs concentrés étant toutefois "maîtrisée" par l'intervention de polymère(s) de forte masse moléculaire, qui garantissent l'homogénéité du produit réticulé puis de l'hydrogel obtenus.

Selon son premier objet, la présente invention concerne donc un procédé de réticulation de hyaluronate de sodium (NaHA) mis en oeuvre en solvant aqueux par action d'une quantité efficace et non excessive d'au moins un agent de réticulation, telle qu'elle assure théoriquement un taux de réticulation défini par le rapport : 100 x (nombre total de fonctions réactives dudit agent de réticulation / nombre total de motifs disaccharidiques des molécules de polymère présentes) compris entre 0.5 et 70%, amélioré en ce qu'il est mis en oeuvre sur un mélange de NaHA intervenant sous la forme de deux masses moléculaires différenciées renfermant plus de 50% en masse de NaHA de faible masse moléculaire moyenne m $\approx 3 \times 10^5$ Da, déterminée à l'aide de la viscosité intrinsèque, et moins de 50%, mais au moins 5% en masse de NaHA de forte masse moléculaire moyenne M $\approx 3 \times 10^6$ Da, déterminée à l'aide de la viscosité intrinsèque.

Ledit mélange renferme bien entendu le(s)dit(s) polymère(s) de faible masse moléculaire en quantité suffisante pour garantir une concentration relativement élevée du milieu réactionnel en polymère(s) et le(s)dit(s) polymère(s) de forte masse moléculaire en quantité suffisante pour garantir une consistance homogène audit polymère réticulé obtenu.

Dans le contexte de la réticulation de ce type de polymère, l'homme du métier comprend que ladite réticulation est mise en oeuvre dans un solvant aqueux basique. De manière générale, ladite réticulation est évidemment mise en oeuvre dans des conditions de pH favorable à la dissolution du polymère en cause.

Dans un tel contexte, ledit mélange réactionnel a avantageusement une viscosité intrinsèque inférieure à 1900 ml/g, i.e. $\Sigma \omega_i [\eta_i]_0 < 1\,900$ ml/g, avec $\omega_i$ : fraction massique de la fraction i de polymère, présentant une viscosité intrinsèque $[\eta_i]_0$, dans le mélange réactionnel. L'homme du métier connaît le paramètre viscosité intrinsèque et n'ignore pas les lois d'additivité dudit paramètre.

La condition énoncée ci-dessus permet d'obtenir un hydrogel monophasique optimisé en référence à ses propriétés de rémanence et d'injectabilité. Elle fixe l'intervention relative des sels de faible (m) et forte (M) masse moléculaire.

Dans le contexte présentement évoqué (NaHA de masses moléculaires m et M), le mélange réactionnel renferme avantageusement plus de 70 % en masse de NaHA de faible masse moléculaire m et donc, logiquement, avantageusement moins de 30 % en masse de NaHA de forte masse moléculaire M.

A titre d'agent de réticulation on peut faire intervenir tout agent connu pour réticuler les polysaccharides et leurs dérivés, par l'intermédiaire de ses fonctions hydroxyles - agent réticulant au moins bifonctionnel, pour assurer la réticulation - et notamment un époxy ou ses dérivés.

On préconise l'intervention d'agents de réticulation, bifonctionnels, seuls ou en mélange. On préconise particulièrement l'intervention de Pépichlorhydrine, du divinylsulfone, du 1,4-bis(2,3-époxypropoxy)butane (ou 1,4-bisglycidyloxybutane ou encore 1,4-butanedioldiglycidyléther ou BDDE), du 1,2-bis(2,3-époxypropoxy)éthylène, du 1-(2,3-époxypropyl)-2,3-époxycyclohexane, d'aldéhydes tels le formaldéhyde, le glutaraldéhyde et le crotonaldéhyde, pris seuls ou en mélange. On préconise tout particulièrement l'intervention du 1,4-bis-(2,3-époxypropoxy)butane ou BDDE.

L'homme du métier saura déterminer la quantité efficace et non excessive d'agent(s) de réticulation à faire intervenir. On préconise d'utiliser une quantité efficace et non excessive telle qu'elle assure théoriquement un taux de réticulation ($\tau$), défini par le rapport

$$\tau = \frac{\text{Nombre total de fonctions réactives dudit agent de réticulation}}{\text{Nombre total de motifs disaccharides des molécules de polymère}} \times 100$$

avantageuseument compris entre 4 et 50 %.

Le procédé de réticulation de l'invention est original de par les formes d'intervention des polymères en cause. Il est sinon mis en oeuvre de façon classique avec au moins un agent de réticulation. On note que ledit agent de réticulation est généralement mis à réagir sur le(s) polymère(s) dissous. Il n'est toutefois nullement exclu qu'il intervienne sur le(s)dit(s) polymère(s) en cours d'hydratation, selon le procédé décrit dans WO-A-02 06 350.

Le réticulat obtenu à l'issue de la mise en oeuvre du procédé de réticulation de l'invention est généralement formulé pour générer l'hydrogel monophasique injectable recherché. Il est si nécessaire préalablement neutralisé. On a vu que la réticulation desdits sels de sodium de l'acide hyaluronique est en effet mise en oeuvre en milieu basique. La formulation est mise en oeuvre dans une solution tamponnée à un pH compatible avec le corps humain (puisque l'hydrogel en cause est généralement prévu pour une injection dans le corps humain) : pH compris entre 6,5 et 7,5 avantageusement entre 7 et 7,4, très avantageusement entre 7,1 et 7,3. Dans ladite solution, le polymère réticulé s'équilibre. Il acquiert également une osmolarité compatible avec celle du corps humain. De façon surprenante, à l'issue cette étape de formulation, les polymères réticulés de l'invention dilués sont des hydrogels <u>mono</u>phasiques.

Selon une variante de mise en oeuvre préférée, on prépare un hydrogel injectable de l'invention, en réticulant un mélange d'au moins un polymère, sel(s) de l'acide hyaluronique (voir ci-dessus), en neutralisant le réticulat obtenu, puis en le formulant à une solution tamponnée à un pH compris entre 7,1 et 7,3, à une concentration comprise entre 10 et 40 mg/g, avantageusement entre 20 et 30 mg/g.

Le procédé de préparation de l'hydrogel monophasique injectable, à partir du polymère réticulé (selon le procédé de réticulation, premier objet de la présente invention) constitue le second objet de la présente invention.

On en vient aux troisième et quatrième objets qui consistent respectivement en le polymère réticulé susceptible d'être obtenu à l'issue de la mise en oeuvre du procédé de réticulation (premier objet) et l'hydrogel monophasique injectable susceptible d'être obtenu par formulation (second objet) dudit polymère réticulé, telle que précisée ci-dessus.

La structure de l'hydrogel monophasique injectable - quatrième objet de la présente invention - est originale. Sa consistance résiste à la dégradation. Cette résistance de l'hydrogel est nettement supérieurs à celle des produits équivalents de l'art antérieur.

L'homme du métier n'ignore pas qu'une des méthodes pour estimer la consistance d'un hydrogel, notamment de ce type, est la mesure du paramètre :

$$\text{tan.delta} = \frac{G''}{G'} = f(\text{fréquence de sollicitation}).$$

Les hydrogels de l'invention ont les débouchés indiqués dans l'introduction du présent texte. Ils s'y révèlent particulièrement performants.

On se propose maintenant d'illustrer l'invention sous ses différents aspects, par les exemples ci-après. On a plus précisément :

- l'exemple 1 qui illustre l'art antérieur (réticulation de NaHA de forte masse moléculaire),
- l'exemple 2 qui illustre les propos énoncés dans l'introduction du présent texte (réticulation de NaHA de faible masse moléculaire),
- les exemples 3 et 4 qui illustrent l'invention (réticulation de NaHA intervenant à différentes quantités relatives à faible et forte masse moléculaire).

On précise préalablement quelques méthodes de mesure, utilisées pour caractériser les produits en cause.

**Mesure de la viscosité intrinsèque**

La viscosité intrinsèque du hyaluronate de sodium (NaHA) (en ml/g) est déterminée conformément à la Pharmacopée Européenne du NaHA (2.2.9), par un viscosimètre capillaire de type Ubelhode.

**Mesure de la force d'éjection** (pas de norme spécifique sur ce test)

L'injectabilité du gel à base de NaHA est déterminée par la mesure de la force nécessaire (en Newton, N) pour éjecter le gel contenu dans une seringue type, à travers une aiguille de 27 G½, à une vitesse de 12,5 mm/min. Les essais ont été effectués sur un appareil de traction Verstatet® commercialisé par la Société Mecmésin.

**Mesure de la rémanence**

La consistance du gel est caractérisée à 25°C par une mesure rhéologique des modules élastique (G') et visqueux (G") en fonction de la fréquence (de 0,05 à 10 Hz) et dans les domaines de déformation constante, au moyen d'un rhéomètre à contrainte imposée (Carrimed CSL 500 de TA Instruments) et d'une géométrie cône/plan 4 cm 2°. Ce rhéomètre est contrôlé et étalonné régulièrement. La dégradation du gel réticulé se traduit par une évolution de sa consistance, que l'on mesure par l'augmentation du paramètre tangente delta (tan.delta=G"/G') en fonction du temps, à la fréquence de 1 Hz. Les gels sont dégradés en étant portés à la température de 93°C. A cette température, on mesure le temps au bout duquel la tan.delta atteint la valeur 0,65 (valeur qui correspond à un état de gel dégradé). Arbitrairement, on a fixé pour le gel de l'exemple 1, un indice de rémanence de 1 (correspondant audit temps). Les valeurs d'indice de rémanence, indiquées pour les autres gels, sont des valeurs relatives.

**Aspect de l'hydrogel**

**Monophasique**

Aspect microscopique : pas de phase liquide apparente - fragmentation fine du gel en facettes
Aspect macroscopique : souple et filant

**Biphasique**

Aspect microscopique : fragments de gels qui baignent dans un milieu liquide peu visqueux.
Aspect macroscopique "purgée" qui fragmente très facilement - pas de cohésion du gel ni d'aspect filant.

EXEMPLE DE REFERENCE

**1 : fibres de haute masse**

On pèse 3,5 g de fibres de hyaluronate de sodium (NaHA), de viscosité intrinsèque = 2 800 ml/g, avec un taux d'humidité de 8,7 %, auquel on ajoute 25,6 g de NaOH à 0,25 N. L'hydratation des fibres dure 2 h, avec une homogénéisation manuelle régulière à la spatule. 0,96 g d'une solution de 1,4-butanedioldiglycidyléther (BDDE) dilué au 1/5ème dans la soude 0,25 N est ajouté au milieu réactionnel suivi d'une homogénéisation mécanique de 15 min avant immersion dans un bain thermostaté à 50°C ± 1°C.

$$R = [BDDE)_0/[NaHA]_0 = 6\ \% \ ; \ [NaHA]_i = 105\ mg/g$$

La réaction dure 2 h. Le réticulat est neutralisé à pH = 7,2 dans une solution de tampon phosphate, puis est dialysé. L'hydrogel obtenu est alors ajusté en concentration ($[NaHA]_f$ = 26 mg/g) et homogénéisé mécaniquement avant d'être conditionné en seringues et stérilisé à l'autoclave par la chaleur humide.

Force d'injection après stérilisation : 25 N

Indice de rémanence de l'hydrogel : 1,0
Hydrogel monophasique

EXEMPLE DE REFERENCE

**2 : fibres de faible masse**

On pèse 1,56 g de fibres de hyaluronate de sodium (NaHA), de viscosité intrinsèque = 600 ml/g, avec un taux d'humidité de 5,5 %, auquel on ajoute 7,15 g de NaOH à 0,25 N. L'hydratation des fibres dure 2 h, avec une homogénéisation manuelle régulière à la spatule. 0,31 g d'une solution de 1,4-butanedioldiglycidyléther (BDDE) dilué au 1/5ème dans la soude 0,25 N est ajouté au milieu réactionnel suivi d'une homogénéisation mécanique de 15 min avant immersion dans un bain thermostaté à 50°C $\pm$ 1°C.

$$R = [BDDE]_0/[NaHA]_0 = 6,8 \% \; ; \; [NaHA]_i = 174 \; mg/g$$

La réaction dure 2 h. Le réticulat est neutralisé à pH = 7,2 dans une solution phosphate, puis est dialysé. L'hydrogel obtenu est alors ajusté en concentration ($[NaHA]_f$ = 26 mg/g) et homogénéisé mécaniquement avant d'être conditionné en seringues et stérilisé à l'autoclave.

Force d'injection après stérilisation : 24 N
Indice de rémanence de l'hydrogel : 6,0
Hydrogel biphasique

EXEMPLE SELON L'INVENTION

**3 : mélange de fibres**

On pèse 0,763 g de fibres de hyaluronate de sodium (NaHA) de viscosité intrinsèque = 600 ml/g avec un taux d'humidité de 5,5 % et 0,237 g de fibres de hyaluronate de sodium de viscosité intrinsèque 2 800 ml/g, avec un taux d'humidité de 9,3 %. Proportion massique dans le mélange : 600/2 800 : 77/23 (w/w).
Le mode opératoire reste identique à celui de l'exemple 2.

$R = [DBBE]_0/[NaHA]_0$ = 7 % ; $[NaHA]_i$ = 140 mg/g ; $[NaHA]_f$ = 26 mg/g
Force d'injection après stérilisation : 15 N
Indice de rémanence de l'hydrogel : 3,6
Hydrogel monophasique

EXEMPLE SELON L'INVENTION

**4 : mélange de fibres**

L'expérience de l'exemple 3 est reproduite en modifiant la proportion massique. Proportion massique dans le mélange : 600/2 800 : 90/10 (w/w)
Le mode opératoire est identique à celui de l'exemple 2.

$R = [BDDE]_0/[NaHA]_0$ = 6,5 % ; $[NaHA]_i$ = 140 mg/g ;
$[NaHA]_f$ = 26 mg/g
Force d'injection après stérilisation : 14 N
Indice de rémanence de l'hydrogel ; 7,7
Hydrogel monophasique

Lesdits exemples sont récapitulés dans le tableau ci-après.

TABLEAU

[NaHA]$_0$ = concentration en NaHA dans le milieu réactionnel à t$_0$

[NaHA]$_f$ = concentration en NaHA dans l'hydrogel final après réaction et dilution avec une quantité qsp de tampon phosphate

G' : module élastique de l'hydrogel final (Pa.s)

G" : module visqueux de l'hydrogel final (Pa.s)                    Rhéomètre Carrimed CSL 500

Tan.delta = G"/G'

$\eta_{int.}$ : viscosité intrinsèque de la fibre de NaHA/Viscosimètre Ubelhode

F : force d'éjection du gel en N à travers une aiguille 27G½/Dynamomètre 100N

| n° | $\eta_{in}$ (ml/g) % = proportion massique dans le mélange | R = $m_{BDDE}/m_{Na}$HA | [NaHA]$_0$ mg/g | [NaHA]$_f$ dans gel final mg/g | Aspect* | G', G", tan.delta (1 Hz) | F$_{ap\ ster}$ 27 G½ | Indice de rémanence |
|---|---|---|---|---|---|---|---|---|
| 1 | (100 %) 2 800 | 6% | 105 | 26 | M | 143/65/0,40 | 25 | 1 |
| 2 | (100 %) 600 | 6,8% | 174 | 26 | B | 1 300/100/0,08 | 24 | 6 |
| 3 | (77 %) 600 + (23 %) 2 800 | 7 | 140 | 26 | M | 262/27/0,10 | 15 | 3,6 |
| 4 | (90 %) 600 + (10 %) 2 800 | 6,5 | 140 | 26 | M | 571/41/0,07 | 14 | 7,7 |

* M = monophasique B = biphasique

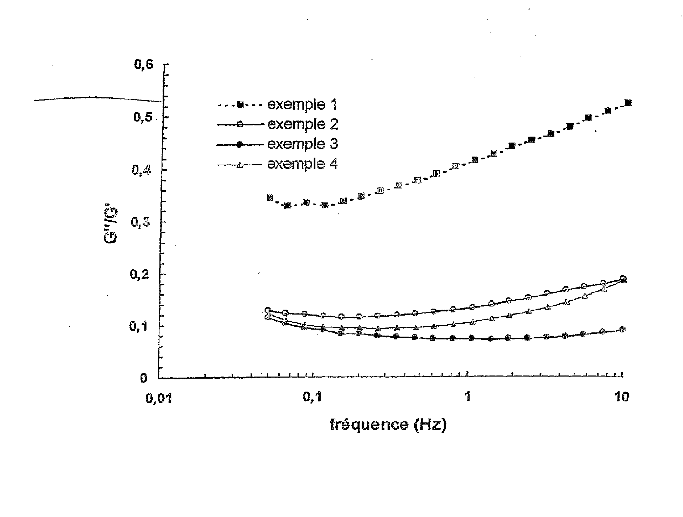

Sur la figure annexée, on a montré pour chacun des quatre hydrogels, préparés selon les exemples 1 à 4, les courbes : Tan.delta = f (fréquence de sollicitation).

Les hydrogels selon 3 et 4) ont un comportement rhéologique différent de celui de l'hydrogel de l'art antérieur (exemple 1).

Ils sont par ailleurs monophasiques et en cela très différents de l'hydrogel de l'exemple 2 (biphasique).

## Revendications

1. Procédé de réticulation de hyaluronate de sodium (NaHA) mis en oeuvre en solvant aqueux par action d'une quantité efficace et non excessive d'au moins un agent de réticulation tel que ladite quantité assure théoriquement un taux de réticulation défini par le rapport: 100 x (nombre total de fonctions réactives dudit agent de réticulation/nombre total de motifs disaccharidiques des molécules de polymère présentes), compris entre 0.5 et 70%, **caractérisé en ce qu'**il est mis en oeuvre :

    - sur un mélange de hyaluronate de sodium intervenant sous la forme de deux masses moléculaires différenciées renfermant:

        - plus de 50% en masse de NaHA de faible masse moléculaire moyenne m $\approx$ 3 x $10^5$ Da, déterminée à l'aide de la viscosité intrinsèque, et
        - moins de 50%, mais au moins 5% en masse de NaHA de forte masse moléculaire moyenne M $\approx$ 3 x $10^6$ Da, déterminée à l'aide de la viscosité intrinsèque.

2. Procédé selon la revendication 1, **caractérisé en ce que** ledit mélange a une viscosité intrinsèque inférieure à 1 900 ml/g.

3. Procédé selon la revendication 1, **caractérisé en ce que** ledit mélange renferme plus de 70% en masse d'au moins un NaHA de ladite faible masse moléculaire moyenne m et moins de 30 % en masse d'au moins un NaHA de ladite forte masse moléculaire moyenne M.

4. Procédé selon l'une quelconque des revendications 1 à 3, **caractérisé en ce que** ledit mélange renferme environ 90 % en masse de NaHA présentant une masse moléculaire moyenne d'environ $3 \cdot 10^5$ Da et environ 10 % en masse de NaHA présentant une masse moléculaire moyenne d'environ $3 \cdot 10^6$ Da.

5. Procédé selon l'une quelconque des revendications 1 à 4, **caractérisé en ce que** ledit agent de réticulation est choisi parmi les agents de réticulation bifonctionnels et leurs mélanges.

6. Procédé selon la revendication 5, **caractérisé en ce que** ledit agent de réticulation est choisi parmi l'epichlorhydrine, le divinylsulfone, le 1,4-bis(2,3-époxypropoxy) butane, le 1,2-bis(2,3-époxypropoxy)éthylène, le 1-(2,3-époxypropyl)-2,3-époxycyclohexane, les aldéhydes, et leurs mélanges.

7. Procédé selon la revendication 5, **caractérisé en ce que** ledit agent de réticulation est le 1,4-bis(2,3-époxypropoxy) butane.

8. Procédé selon l'une quelconque des revendications 1 à 7, **caractérisé en ce que** ladite quantité efficace et non excessive d'au moins un agent de réticulation assure théoriquement un taux de réticulation, défini par le rapport: 100 x (nombre total de fonctions réactives dudit agent de réticulation/nombre total de motifs disaccharidiques des molécules de polymère présentes), compris entre 4 et 50 %.

9. Procédé de préparation d'un hydrogel monophasique injectable de polymère réticulé de NaHA **caractérisé en ce qu'**il comprend:

    - la réticulation d'un mélange selon l'une quelconque des revendications 1 à 8,
    - la formulation dudit mélange réticulé, neutralisé si nécessaire, dans une solution tamponnée à un pH compris entre 6,5 et 7,5.

10. Procédé de préparation d'un hydrogel monophasique injectable de polymère réticulé de NaHA selon la revendication 9, **caractérisé en ce qu'**il comprend la formulation dudit mélange réticulé, neutralisé si nécessaire, dans une solution tamponnée à un pH compris entre 7 et 7,4.

11. Procédé de préparation d'un hydrogel monophasique injectable de polymère réticulé de NaHA selon la revendication 9, **caractérisé en ce qu'**il comprend la formulation dudit mélange réticulé, neutralisé si nécessaire, dans une solution tamponnée à un pH compris entre 7,1 et 7,3.

12. Procédé selon l'une quelconque des revendications 9-11, **caractérisé en ce qu'**il comprend:

- la réticulation d'un mélange selon l'une quelconque des revendications 1 à 8;
- la formulation dudit mélange réticulé, neutralisé, dans une solution tamponnée à un pH compris entre 7,1 et 7,3, à une concentration comprise entre 10 et 40 mg/g.

13. Procédé selon la revendication 12, **caractérisé en ce que** la formulation dudit mélange réticulé, neutralisé, dans une solution tamponnée à un pH compris entre 7,1 et 7,3 s'effectue, à une concentration comprise entre 20 et 30 mg/g.

14. Polymère réticulé susceptible d'être obtenu à l'issue de la mise en oeuvre d'un procédé de réticulation selon l'une quelconque des revendications 1 à 8.

15. Hydrogel monophasique injectable susceptible d'être obtenu à l'issue de la mise en oeuvre d'un procédé de préparation selon l'une des revendications 9 à 13.

**Patentansprüche**

1. Verfahren zur Vernetzung von Natriumhyaluronat (NaHA), das in einem wässrigen Lösungsmittel durch Einwirkung einer wirksamen und nicht überschüssigen Menge wenigstens eines Vernetzungsmittels so durchgeführt wird, dass die Menge theoretisch einen Vernetzungsgrad zwischen 0,5 und 70% sicherstellt, der durch das Verhältnis:

```
100 x (Gesamtanzahl der reaktionsfähigen Funktionen des
Vernetzungsmittels/Gesamtanzahl der wiederkehrenden
Disaccharideinheiten der vorhandenen Polymermoleküle)
definiert wird,
```

**dadurch gekennzeichnet, dass**:

- es mit einer Mischung von Natriumhyaluronat in Form von zwei unterschiedlichen Molekularmassen durchgeführt wird, enthaltend:

- mehr als 50 Masse% NaHA mit niederem durchschnittlichen Molekulargewicht $m \approx 3 \times 10^5$ Da, das mit Hilfe der Grenzviskosität bestimmt wird, und
- weniger als 50 Masse%, aber mehr als 5 Masse% NaHA mit hohem durchschnittlichen Molekulargewicht $M \approx 3 \times 10^6$ Da, das mit Hilfe der Grenzviskosität bestimmt wird.

2. Verfahren gemäß Anspruch 1, **dadurch gekennzeichnet, dass** die Mischung eine Grenzviskosität von weniger als 1.900 ml/g aufweist.

3. Verfahren gemäß Anspruch 1, **dadurch gekennzeichnet, dass** die Mischung mehr als 70 Masse% von wenigstens einem NaHA mit dem niederen durchschnittlichen Molekulargewicht m und weniger als 30 Masse% von wenigstens einem NaHA mit dem hohen durchschnittlichen Molekulargewicht M enthält.

4. Verfahren gemäß einem der Ansprüche 1-3, **dadurch gekennzeichnet, dass** die Mischung etwa 90 Masse% NaHA, das ein durchschnittliches Molekulargewicht von etwa $3 \cdot 10^5$ Da aufweist, und etwa 10 Masse% NaHA, das ein durchschnittliches Molekulargewicht von etwa $3 \cdot 10^6$ Da aufweist, enthält.

**5.** Verfahren gemäß einem der Ansprüche 1-4, **dadurch gekennzeichnet, dass** das Vernetzungsmittel aus bifunktionellen Vernetzungsmitteln und ihren Mischungen ausgewählt ist.

**6.** Verfahren gemäß Anspruch 5, **dadurch gekennzeichnet, dass** das Vernetzungsmittel aus Epichlorhydrin, Divinylsulfon, 1,4-Bis(2,3-epoxypropoxy)butan, 1,2-Bis(2,3-epoxypropoxy)ethylen, 1-(2,3-Epoxypropyl)-2,3-epoxycyclohexan, Aldehyden und ihren Mischungen ausgewählt ist.

**7.** Verfahren gemäß Anspruch 5, **dadurch gekennzeichnet, dass** das Vernetzungsmittel 1,4-Bis(2,3-epoxypropoxy)butan ist.

**8.** Verfahren gemäß einem der Ansprüche 1-7, **dadurch gekennzeichnet, dass** die wirksame und nicht überschüssige Menge wenigstens eines Vernetzungsmittels theoretisch einen Vernetzungsgrad zwischen 4 und 50% sicherstellt, der durch das Verhältnis: 100 x (Gesamtanzahl der reaktionsfähigen Funktionen des Vernetzungsmittels/Gesamtanzahl der wiederkehrenden Disaccharideinheiten der vorhandenen Polymermoleküle) definiert wird.

**9.** Verfahren zur Herstellung eines einspritzbaren einzelphasigen Hydrogels aus vernetztem Polymer aus NaHA, **dadurch gekennzeichnet, dass** es umfasst:

- die Vernetzung einer Mischung gemäß einem der Ansprüche 1-8,
- die Formulierung der Vernetzungsmischung, wenn notwendig neutralisiert, in einer auf einen pH zwischen 6,5 und 7,5 gepufferten Lösung.

**10.** Verfahren zur Herstellung eines einspritzbaren einzelphasigen Hydrogels aus vernetztem Polymer aus NaHA gemäß Anspruch 9, **dadurch gekennzeichnet, dass** es die Formulierung der Vernetzungsmischung, wenn notwendig neutralisiert, in einer auf einen pH zwischen 7 und 7,4 gepufferten Lösung umfasst.

**11.** Verfahren zur Herstellung eines einspritzbaren einzelphasigen Hydrogels aus vernetztem Polymer aus NaHA gemäß Anspruch 9, **dadurch gekennzeichnet, dass** es die Formulierung der Vernetzungsmischung, wenn notwendig neutralisiert, in einer auf einen pH zwischen 7,1 und 7,3 gepufferten Lösung umfasst.

**12.** Verfahren gemäß einem der Ansprüche 9-11, **dadurch gekennzeichnet, dass** es umfasst:

- die Vernetzung einer Mischung gemäß einem der Ansprüche 1-8;
- die Formulierung der neutralisierten Vernetzungsmischung in einer auf einen pH zwischen 7,1 und 7,3 gepufferten Lösung in einer Konzentration zwischen 10 und 40 mg/g.

**13.** Verfahren gemäß Anspruch 12, **dadurch gekennzeichnet, dass** die Formulierung der neutralisierten Vernetzungsmischung in einer auf einen pH zwischen 7,1 und 7,3 gepufferten Lösung in einer Konzentration zwischen 20 und 30 mg/g erfolgt.

**14.** Vernetztes Polymer, das nach einem gemäß einem der Ansprüche 1-8 durchgeführten Verfahren zur Vernetzung erhältlich ist.

**15.** Einspritzbares einzelphasiges Hydrogel, das nach einem gemäß einem der Ansprüche 9-13 durchgeführten Herstellungsverfahren erhältlich ist.

**Claims**

**1.** Process for cross-linking of sodium hyaluronate (NaHA) implemented in aqueous solvent by action of an effective and non-excessive quantity of at least one cross-linking agent such that the said quantity theoretically provides a cross-linking rate defined by the ratio: 100 x (total number of reactive functions of the said cross-linking agent/total number of disaccharide structures of the polymer molecules present), of between 0.5 and 70%, **characterised in that** it is implemented:

- on a mixture of sodium hyaluronate taking part in the form of two differentiated molecular masses containing:

- more than 50% by mass of NaHA of low mean molecular mass m $\sim$ 3 x $10^5$ Da, determined by means of the intrinsic viscosity, and

- less than 50%, but at least 5% by mass of NaHA of high mean molecular mass M ~ 3 x 10$^6$ Da, determined by means of the intrinsic viscosity.

**2.** Process as described in claim 1, **characterised in that** the said mixture has an intrinsic viscosity lower than 1 900 ml/g.

**3.** Process as described in claim 1, **characterised in that** the said mixture contains more than 70% by mass of at least one NaHA of the said low mean molecular mass m and less than 30% by mass of at least one NaHA of the said high mean molecular mass M.

**4.** Process as described in any one of claims 1 to 3, **characterised in that** the said mixture contains approximately 90% by mass of NaHA presenting a mean molecular mass of approximately 3⊙10$^5$ Da and approximately 10% by mass of NaHA presenting a mean molecular mass of approximately 3⊙10$^6$ Da.

**5.** Process as described in any one of claims 1 to 4, **characterised in that** the said cross-linking agent is selected from bifunctional cross-linking agents and their mixtures.

**6.** Process as described in claim 5, **characterised in that** the said cross-linking agent is selected from epichlorhydrin, divinylsulphone, 1,4-bis(2,3-epoxypropoxy)butane, 1,2-bis(2,3-epoxypropoxy)ethylene, 1-(2,3-epoxypropyl)-2,3-epoxycyclohexane, aldehydes and their mixtures.

**7.** Process as described in claim 5, **characterised in that** the said cross-linking agent is 1,4-bis(2,3-epoxypropoxy)butane.

**8.** Process as described in any one of claims 1 to 7, **characterised in that** the said effective and non-excessive quantity of at least one cross-linking agent theoretically provides a cross-linking rate, defined by the ratio: 100 x (total number of reactive functions of the said cross-linking agent/total number of disaccharide structures of the polymer molecules present) of between 4 and 50%.

**9.** Process for preparation of an injectable monophase hydrogel of cross-linked polymer of NaHA **characterised in that** it comprises:

- the cross-linking of a mixture as described in any one of claims 1 to 8,
- the formulation of the said cross-linked mixture, neutralised if necessary, in a solution buffered to a pH of between 6.5 and 7.5.

**10.** Process for preparation of an injectable monophase hydrogel of cross-linked polymer of NaHA as described in claim 9, **characterised in that** it comprises the formulation of the said cross-linked mixture, neutralised if necessary in a solution buffered to a pH of between 7 and 7.4.

**11.** Process for preparation of an injectable monophase hydrogel of cross-linked polymer of NaHA as described in claim 9, **characterised in that** it comprises the formulation of the said cross-linked mixture, neutralised if necessary, in a solution buffered to a pH of between 7.1 and 7.3.

**12.** Process as described in any one of claims 9-11, **characterised in that** it comprises:

- the cross-linking of a mixture as described in any one of claims 1 to 8;
- the formulation of the said cross-linked mixture, neutralised, in a solution buffered to a pH of between 7.1 and 7.3, at a concentration of between 10 and 40 mg/g.

**13.** Process as described in claim 12, **characterised in that** the formulation of the said cross-linked mixture, neutralised in a solution buffered to a pH of between 7.1 and 7.3, is carried out at a concentration of between 20 and 30 mg/g.

**14.** Cross-linked polymer obtainable following the implementation of a cross-linking process as described in any one of claims 1 to 8.

**15.** Injectable monophase hydrogel obtainable following the implementation of a preparation process as described in one of claims 9 to 13.

**EP 2 289 945 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0466300 A
- WO 9633751 A
- WO 0001428 A
- WO 9835639 A
- WO 9835640 A
- US 4716154 A
- WO 0205753 A
- WO 0206350 A